# EUROPÄISCHE PATENTSCHRIFT

(11) **EP 2 366 376 B1**
(45) Veröffentlichungstag und Bekanntmachung des Hinweises auf die Patenterteilung: **13.01.2016**
(21) Anmeldenummer: 10002293.8
(22) Anmeldetag: 05.03.2010
(51) Int. Cl.: A61K 8/34, A61K 8/37, A61K 8/44, A61K 8/60, A61K 8/92, B01F 17/28, B01F 17/56, B01F 17/36, A61Q 13/00

(54) **SOLUBILISATORZUBEREITUNG**
SOLUBILISER PREPARATION
PRÉPARATION SOLUBILISANTE

(43) Veröffentlichungstag der Anmeldung: 21.09.2011
(73) Patentinhaber: Dr. Straetmans Chemische Produkte GmbH, 22143 Hamburg (DE)
(72) Erfinder: Jänichen, Jan, 22149 Hamburg (DE); Petersen, Wilfried, 22143 Hamburg (DE); Salmina-Petersen, Manuela, 22143 Hamburg (DE); Nazarova, Daria, 22159 Hamburg (DE); Scholze, Jessica, 21423 Winsen/Luhe (DE)
(74) Vertreter: Glawe, Delfs, Moll

(56) Entgegenhaltungen:
- EP-A1- 1 250 916
- EP-A1- 1 700 618
- EP-A2- 1 502 644
- WO-A1-2006/087156
- DE-A1-102005 051 864
- JP-A- 2 067 247
- Anonymous: "Resassol VPF"[Online] 24. August 2009 (2009-08-24), Seiten 1-2, XP002597085 kinetiktech Gefunden im Internet: URL:http://www.kinetiktech.com/docs/news/0 907.pdf> [gefunden am 2010-08-16]

## Beschreibung

Die vorliegende Erfindung betrifft Solubilisatorzubereitungen gemäß dem Oberbegriff des Anspruchs 1.

Unter Solubilisierung versteht man das klare Auflösen eines lipophilen Stoffes in einer wässrigen Matrix unter Verwendung oberflächenaktiver Hilfsmittel, den sogenannten Solubilisatoren.

Bei den Solubilisatoren handelt es sich um eine Gruppe amphiphiler Substanzen, welche in der Lage sind, lipophile Substanzen in wässrigen Systeme in micellaren Strukturen einzubinden, die eine so geringe Größe besitzen, dass sie durchscheinendes Licht nicht streuen. Das entstehende Solubilisat erscheint dadurch transparent. Solubilisate bestehen dabei im Gegensatz zu Produkten der Körperreinigung meist zu über 90% aus Wasser oder wässrigen Lösungen und sollten optimalerweise nicht schäumen. Anwendungen für Solubilisate in der Kosmetikindustrie sind beispielsweise Tonics, Rasierwässer, Mundwässer, Duftwässer aber auch transparente Deodorantien.

Zur Entfaltung ihrer solubilisierenden Wirkung benötigen Solubilisatoren einen großen hydrophilen Molekülteil und einen verhältnismäßig kleinen lipophilen Molekülteil. In dem von Griffin beschrieben HLB-System zur Klassifizierung oberflächenaktiver Substanzen besitzen Solubilisatoren daher Werte dicht am Maximalwert von 20 (Griffin, W. C.: Classification of surface active agents by HLB, J. Soc. Cosmet. Chem. 1, 1949).

Dem Fachmann ist darüber hinaus bekannt, dass zur Herstellung transparenter Solubilisate die zu solubilisierende, lipophile Substanz zunächst homogen mit dem Solubilisator vermischt werden sollte, bevor sie mit der wässrigen Phase verdünnt wird. Das transparente Solubilisieren von lipophilen Substanzen in stark wässriger Verdünnung schlägt hingegen in der Regel fehl.

Da es sich bei den zu solubilisierenden Substanzen meist um Duft- oder Aromastoffe handelt, sind solche Solubilisatoren bevorzugt, welche die geruchlichen oder geschmacklichen Eigenschaften des Solubilisats nicht oder nur gering beeinflussen. Vor dem Hintergrund eines wachsenden Umweltbewusstseins sind darüber hinaus solche Solubilisatoren besonders wünschenswert, die aus erneuerbaren Rohstoffen gewonnen werden können. Da in vielen Fällen zusätzlich Wirkstoffe mit pH-abhängiger Aktivität in Solubilisate eingearbeitet werden, entfaltet ein vielseitiger Solubilisator seine Eigenschaften optimalerweise über einen breiten pH-Bereich.

Zur Solubilisierung von lipophilen Substanzen wie Ölen, Aromen oder Parfumstoffen in wässrigen Matrices kosmetischer Formulierungen sind dem Fachmann vor allem polyethoxylierte Derivate des hydrierten Rizinusöls (PEG-40 Hydrogenated Castor Oil; z.B. Cremophor RH 40) oder polyethoxylierte Sorbitanester z.B. Tween 20 bekannt. Diese PEG-haltigen Solubilisatorsysteme weisen zwar sehr gute, pH-unabhängige solubilisierende Eigenschaften aus, basieren jedoch zumindest teilweise auf Rohstoffen fossilen Ursprungs. Darüber hinaus stehen polyethoxylierte Verbindungen im Verdacht, die Hautbarriere leicht und unbeschadet zu penetrieren und dadurch Schadstoffe durch die Haut zu transportieren.

Aus diesem Grund und im Zuge eines wachsenden Trends zur Verwendung von Rohstoffen aus nachwachsenden Quellen wurde daher in den vergangenen Jahren nach PEG-freien Alternativen zu den o.g. Solubilisatoren gesucht.

Die derzeit auf dem Markt erhältlichen alternativen Systeme sind aus den Stoffgruppe alkylierter Glucoside, acylierter Aminosäuren, Polyglycerinester oder deren Gemische aufgebaut. Beispielhaft seien hier z.B. das Caprylyl/Capryl Glucoside oder das Polyglycerin-10 Laurate genannt. Während diese Rohstoff zwar den Anforderungen nachkommen, aus nachwachsenden Rohstoffe herstellbar zu sein, sind deren Solubilisierungskapazität in vielen Fällen jedoch unzureichend und transparente Solubilisate nur begrenzt herstellbar.

Eine Verbesserung der Solubilisierungskapazität eines Alkylglucosids verspricht dessen Kombination mit einem Polyglycerinester und einem Acylglutamat. Dieses Konzept wird unter dem Markennamen Ressasol VPF vertrieben. Nachteil dieser Mischung ist der stark eingeschränkte pH-Bereich der Wirksamkeit von > 7,0.

Acyliertes Prolin (INCI: Cocoyl Prolin), welches unter dem Handelsnamen Natisol angeboten wird, zeigt bei pH-Werten > 6,0 gute Solubilisierungseigenschaften, diese lassen jedoch bei niedrigeren pH-Werten schnell nach.

EP 1 211 258 B1 beschreibt Herstellung und Verwendung von Mischungen von aus Fuselalkoholen hergestellten Alkylpolyglycosiden als Solubilisatorzusatz. Die erfindungsgemäßen Glycoside sind dabei vorzugsweise aus den Pentoseeinheiten Xylose und Arabinose aufgebaut und wurden mit Isoamylalkohol glycosiliert. Sie sind unter dem Markennamen Easysurf kommerziell verfügbar.

Gemäß dieser Patentschrift kann das Solubilisierungsverhalten dieser Mischungen durch Zusatz nicht-ionischer, anionischer oder kationischer Tenside weiter verbessert werden. Als besonders gut geeignet werden Gemische mit Alkylglycosiden erwähnt, welche im Patent EP0699472 B1 beschrieben sind.

Die aus Fuselalkoholen erfindungsgemäß hergestellten Alkylpolyglycoside und deren Mischungen weisen jedoch einen starken Eigengeruch auf, der aufgrund der eingeschränkten Solubilisierungskapazität auch in Mischungen mit weiteren Tensiden, den Geruch des Solubilisats signifikant beeinflusst.

Die Forderung nach einem PEG-freien, aus nachwachsenden Rohstoffen herstellbaren Solubilisatorsystem, welches zuverlässig und über einen breiten pH-Bereich eine möglichst große Anzahl verschiedener lipophiler Substanzen transparent in einer wässrigen Matrix zu solubilisieren in der Lage ist und dabei den Geruch des Solubilisats nicht beeinträchtigt, kann mit Hilfe des derzeitigen Standes der Technik als nicht befriedigend gelöst gelten.

Die erfindungsgemäße Aufgabe bestand demnach in der Suche nach Solubilisatorsystemen, welche diese genannten Defizite des Standes der Technik beheben können.

Die Lösung dieser Aufgabe ist im Anspruch 1 angegeben; vorteilhafte Ausführungsformen finden sich in den Unteransprüchen.

Überraschenderweise wurde gefunden, dass die Einarbeitung eines Benetzmittels definierten Molekulargewichts und HLB-Wertes in eine Vielzahl existierender Solubilisatorsysteme zu einer erheblichen Verbesserung derer Solubilisierungsleistung führt und dadurch Solubilisatorsysteme bereitgestellt werden können, welche die erfindungsgemäße Aufgabe zu lösen im Stande sind.

Die erfindungsgemäßen Solubilisatorgemische zeichnen sich gegenüber dem Stand der Technik durch ihre PEG-Freiheit, in Kombination mit einem stark verbesserten und pHunabhängigen Solubilisierungsvermögen aus. PEG-frei bedeutet im Rahmen der Erfindung, dass Polyethylenglykole im der Solubilisatorzubereitung nicht oder allenfalls als Verunreinigung enthalten sind.

Die vorliegende Erfindung betrifft somit PEG-freie Solubilisatorzubereitungen zur transparenten Solubilisierung von lipophilen Substanzen in wässrigen Medien, enthaltend ein oder mehrere Tenside ausgewählt aus den folgenden Gruppen:
a) Alkylpolyglycosiden der allgemeinen Formel R-O-(A)ⱼ-(B)ₖ-(C)ₗ-(D)ₘ-(E)ₙ, wobei R einen linearen oder verzweigten Alkylrest mit einer Kettenlänge von 4 - 12 Kohlenstoffatomen darstellt, A, B, C, D und E unabhängig voneinander Pentose- oder Hexose-Zuckereinheiten repräsentieren und j, k, l, m, n unabhängig voneinander Werte zwischen 0 und 1 annehmen können, wobei die Summe aller Werte j, k, l, m, n mindestens 1 beträgt;
b) Polyglycerinestern gebildet aus einer linearen oder verzweigten, gesättigten oder ungesättigten Fettsäure einer Kettenlänge von 8 - 20 Kohlenstoffatomen und einem Polymerisierungsgrad von n = 2 - 10;
c) N-acylierte Aminosäuren der allgemeinen Formel R'-CO-AS, wobei R' einen linearen oder verzweigten, gesättigten oder ungesättigten Alkylrest von 5 - 11 Kohlenstoffatomen repräsentiert und AS eine der in der Natur vorkommenden Aminosäuren oder deren Salze oder Ester;
   wobei der Gesamtanteil der Tenside a) + b) + c) an der Solubilisatorzubereitung mindestens 50% beträgt, sowie zusätzlich
d)3 und 10%, vorzugsweise zwischen 4 und 8% eines oder mehrerer Benetzmittel mit einem Molekulargewicht von 100 - 250 und einem HLB-Wert nach Griffin von 6 - 12, ausgewählt aus der Gruppe der Monoglyceride von Fettsäuren mit einer Kettenlänge von 6 bis 10 Kohlenstoffatomen oder der 1,2-Alkandiole mit einer Kettenlänge von 6 bis 10 Kohlenstoffatomen.

Die Tenside können als wässrige Konzentrate vorliegen. Sämtliche Prozentangaben in dieser Patentanmeldung sind Gewichtsprozente.

Die erfindungsgemäßen Solubilisatorzusätze unterscheiden sich in ihrer Zusammensetzung dabei von fertigen Produkten zur Körperreinigung durch ihren hohen Tensidgehalt, vor allem aber durch den hohen Gehalt des Benetzmittels. Ein Zusatz von 3-10% der erfindungsgemäßen Benetzmittel zu einem Körperpflegeprodukt würde dessen Anwendungseigenschaften erheblich beeinträchtigen und daher vom Fachmann nicht in Betracht gezogen werden.

Aus der Gruppe a) der Alkylglycoside, die erfindungsgemäß verwendet werden können, sind solche Systeme hervorzuheben, welche vorwiegend aus den Pentoseeinheiten Xylose und Arabinose aufgebaut sind. Bevorzugt sind solche Systeme, die in den Patentschriften EP1211258 B1 und EP0699472 B1 beschrieben sind.

Aus der Gruppe b) der Polyglycerinester, die erfindungsgemäß verwendet werden können, sind solche Systeme hervorzuheben, die einen HLB-Wert nach Griffin von 11 - 16 besitzen. Besonders bevorzugt ist dabei das Polyglycerin-5 Oleate.

Aus der Gruppe c) der N-acylierten Aminosäuren, die erfindungsgemäß verwendet werden können, sind die aus Kokosfettsäuren und Prolin und oder Glutaminsäure gebildeten Vertreter sowie deren Salze hervorzuheben. Besonders bevorzugt ist dabei die Cocoyl Glutaminsäure sowie deren Salze.

Aus der Gruppe der Benetzmittel sind besonders geeignet und damit besonders bevorzugt im Sinne der vorliegenden Erfindung das Glyceryl Caprylate und/oder das Caprylyl Glycol

Ganz besonders wirksame Solubilisatorzusätze im Sinne der vorliegenden Erfindung enthalten
- 30 - 40 %: Caprylyl/Capric Wheat Bran / Straw Glycosides
- 10 - 20 %: Fusel Wheat Bran / Straw Glycosides
- 5 - 15 %: Polyglyceryl-5 Oleate
- 5 - 15 %: Disodium Cocoyl Glutamate
- 3 - 10%: Glyceryl Caprylate
oder
- 30 - 40 %: Caprylyl/Capric Wheat Bran / Straw Glycosides
- 10 - 20 %: Fusel Wheat Bran / Straw Glycosides
- 5 - 15 %: Polyglyceryl-5 Oleate
- 5 - 15 %: Disodium Cocoyl Glutamate
- 3 - 10%: Caprylyl Glycol

Diese sind daher besonders bevorzugt im Sinne der vorliegenden Erfindung.

Die durch die erfindungsgemäßen Mischungen hergestellten Solubilisate zeichnen sich durch eine hohe Stabilität aus, die auch die Erniedrigung des pH-Wertes nicht beeinträchtigt wird.

Gegenstand der Erfindung ist ferner ein Verfahren zur Herstellung eines Solubilisats. Bei diesem Verfahren werden die zu solubilisierenden lipophilen Komponenten zunächst mit einer erfindungsgemäßen Solubilisatorzubereitung homogen zum Erhalt einer transparenten Mischung verrührt. Um hier eine gute Solubilisierungsleistung zu erzielen, ist es erforderlich, dass alle Komponenten des erfindungsgemäßen Solubilisators, insbesondere das Benetzmittel, mit der lipophilen Komponente homogen verrührt werden, bevor im nächsten Schritt Wasser bzw. eine wässrige Phase zugegeben wird. Es ist beispielsweise nicht möglich, zunächst die Tenside mit der zu solubilisierenden Komponente in wässriger Lösung zu verrühren und anschließend das Benetzmittel zuzugeben.

Gegenstand der Erfindung ist ferner die Verwendung eines erfindungsgemäß hergestellten Solubilisats (Mischung aus Solubilisatorzubereitung und lipophiler Komponente homogen verrührt nach Anspruch 8) zur Herstellung eines bzw. in einem Produkt zur Reinigung, Beduftung, Desodorierung oder Pflege der Haut, der Schleimhaut und/oder der Haare.

Die Erfindung wird nachfolgend anhand von Beispielen und Vergleichsbeispielen erläutert.

Im Rahmen der Untersuchungen zur Lösung der erfindungsgemäßen Aufgabe wurde eine Reihe kommerziell verfügbarer, PEGfreier Solubilisatorzusätze ausgewählt und deren Solubilisierungsvermögen getestet. Hierzu wurden verschiedene verfügbare Parfümöle sowie Esteröle und lipophile Aktivstoffe ausgewählt und versucht, diese mit Hilfe der zu testenden Solubilisatoren transparent in Wasser zu lösen. Als Referenzsubstanz wurde PEG-Hydrogenated Castor Oil mitgetestet. Die Versuche wurden wie folgt durchgeführt:
- Menge (g): lipophile Komponente (gemäß Tabelle 1)
- Menge (g): Solubilisator (variabel)
- auf 100 g: Wasser

Eine definierte Menge der lipophilen Komponente wurde mit verschiedenen Mengen des zu testenden Solubilisatorzusatzes homogen vermischt und anschließend auf 100% mit Wasser aufgefüllt. Die auf diese Weise hergestellten Solubilisate wurden auf ihre Transparenz und Stabilität hin beurteilt.

Bei den Zusätzen 2 und 3 wurden dabei die vom Hersteller empfohlenen pH-Bereiche berücksichtigt.

Tabelle 1 gibt die zur transparenten und stabilen Solubilisierung notwendigen Konzentrationen der getesteten Solubilisatorzusätze des Standes der Technik wieder (nicht erfindungsgemäße Vergleichsversuche). Es zeigt sich, dass die zur transparenten Solubilisierung notwendigen Konzentrationen in Abhängigkeit der lipophilen Komponente und des Solubilisatorsystems stark variieren. Einige Systeme waren nicht in der Lage, transparente Solubilisate herzustellen. Die vom Hersteller beschrieben pH-Restriktionen wurden bestätigt. Die Solubilisate der Zusätze 2 und 3 trübten bei Erniedrigung des pH- Wertes ein.

Für die in der Patentschrift EP1211258 B1 genannten Alkylpolyglycoside konnte gezeigt werden, dass der Zusatz von Polyglycerinestern und Acylglutamaten bereits zu einer Verbesserung der Solubilisierungsleistung führt (Zusätze 4-6).

**Tabelle 1: Ergebnisse der Solubilisierungsleistung kommerziell verfügbarer Solubilisatorzubereitungen**

| Konz % | Lipophile Komponente | Lieferant | % Solubilisator | | | | | |
|---|---|---|---|---|---|---|---|---|
| | | | 1 | 2 | 3 | 4 | 5 | 6 |
| 0,5 | Aqua Power | Drom | 3,5 | 3,0 | trüb | 7,5 | 7,0 | 3,0 |
| 0,5 | Body Firm | Drom | 1,0 | 3,5 | 5,0 | 1,8 | 1,7 | 2,5 |
| 0,5 | Sugar Ray | Symrise | 1,0 | 2,5 | 4,0 | 4,5 | 4,5 | 1,6 |
| 0,5 | Leafs in the trees | Symrise | 0,8 | 2,5 | 4,0 | 3,0 | 2,7 | 2,6 |
| 0,5 | Orange Ingwer | Frey & Lau | 3,5 | 4,8 | trüb | 5,5 | 5,3 | 5,0 |
| 0,5 | Priceless Natura | Düllberg | 2,8 | 3,5 | trüb | 5,5 | 5,0 | 3,5 |
| 0,5 | Isoamyl Laurate | Dr. Straetmans | 7,0 | trüb | trüb | trüb | 9,0 | trüb |
| 1,0 | PCA-Glyceryl Oleate | Dr. Straetmans | trüb | 10,0 | trüb | trüb | 4,5 | 4,5 |
| 0,1 | Tocopherol | Dr. Straetmans | 1,3 | Trüb | trüb | trüb | 2,0 | trüb |
| 0,1 | Bisabolol | Merck | 1,0 | 1,8 | trüb | trüb | 3,5 | 1,3 |
| Durchschnittliche Solubilisierungskonzentration | | | 0,9 | 2,8 | 4,3 | 3,1 | 3,0 | 2,2 |

| | | | | | | | | |
|---|---|---|---|---|---|---|---|---|
| 1 PEG-40 Hydrogenated Castor Oil (Cremophor RH 40) 2 Cocoyl Proline, Aqua (pH = 6,0) (Natisol) 3 Polyglyceryl-4 Caprylate, Decyl Glucoside, Sodium Lauroyl Glutamate, Diglycerin, Aqua Ressasol VPF (pH> 7,0) 4 66,5% Fusel Wheat Bran / Straw Glycosides, Aqua (Easysurf 6505) 33,5% Caprylyl/Capric Wheat Bran / Straw Glycosides, Aqua (Easysurf 6781) 5 61,5% Fusel Wheat Bran / Straw Glycosides (Easysurf 6505) 28,5% Caprylyl/Capric Wheat Bran / Straw Glycosides (Easysurf 6781) 10 % Polyglyceryl-5 Oleate 6 50% Fusel Wheat Bran / Straw Glycosides (Easysurf 6505) 25% Caprylyl/Capric Wheat Bran / Straw Glycosides (Easysurf 6781) 10% Polyglyceryl-5 Oleate, 10% Disodium Cocoyl Glutamate | | | | | | | | |

Zur vergleichenden Beurteilung der Solubilisierungsleistung der getesteten Systeme wurde eine durchschnittliche Solubilisierungskonzentration errechnet. Diese ergab sich aus der durchschnittlichen Konzentration, welche notwendig war, die Parfümöle "Body Firm", "Sugar Ray" und "Leafs in the Treas" transparent zu solubilisieren. Diese Parfümöle wurden ausgewählt, da nur sie von allen Solubilisatorzusätzen transparent solubilisiert werden konnten. Die errechneten Werte der durchschnittlichen Solubilisierungskonzentration finden sich in Tabelle 2.

**Tabelle 2: Durchschnittliche Solubilisierungskonzentration der in Tabelle 1 getesteten Solubilisatorsysteme**

| Konz % | Lipophile Komponente | Lieferant | % Solubilisator | | | | | |
|---|---|---|---|---|---|---|---|---|
| | | | 1 | 2 | 3 | 4 | 5 | 6 |
| 0,5 | Body Firm | Drom | 1,0 | 3,5 | 5,0 | 1,8 | 1,7 | 2,5 |
| 0,5 | Sugar Ray | Symrise | 1,0 | 2,5 | 4,0 | 4,5 | 4,5 | 1,6 |
| 0,5 | Leafs in the trees | Symrise | 0,8 | 2,5 | 4,0 | 3,0 | 2,7 | 2,6 |
| | | | | | | | | |
| Durchschnittliche Solubilisierungskonzentration | | | 0,9 | 2,8 | 4,3 | 3,1 | 3,0 | 2,2 |

Unter dem Begriff Benetzmittel werden amphiphile Moleküle zusammengefasst, die eine ausgeprägte Oberflächenaktivität aufweisen, deren Molekulargewicht jedoch deutlich unter denen klassischer Tenside liegt. Als besonders effektiv haben sich in dieser Hinsicht amphiphile Moleküle mit einem Molekulargewicht von 100 - 250 g/mol und einem HLB nach Griffin von 6 - 12 herausgestellt. Beispiele derartiger Benetzmittel findet man in der Gruppe der Monoglyceride von Fettsäuren mit einer Kettenlänge von 6 - 10 Kohlenstoffatomen oder der 1,2-Alkandiole mit einer Kettenlänge von 6 bis 10 Kohlenstoffatomen.

Auch besitzen diese Benetzmittel antimikrobielle Eigenschaften, welche Sie in einer kosmetischen Formulierung vorteilhaft ausbilden können.

Somit eignen sich Benetzmittel zur Erniedrigung von Grenzflächenspannung zwischen einer wässrigen und einer lipophilen Phase. Solubilisierende Eigenschaften zum klaren Auflösen unpolarer Komponenten in Wasser besitzen Benetzmittel hingegen nicht.

Umso überraschender wurde nun gefunden, dass durch den Zusatz von 3 - 10% eines Benetzmittels die solubilisierende Wirkung der oben getesteten Mischungen erheblich verbessert werden konnte.

In Tabelle 3 (erfindungsgemäße Beispiele) sind die zur transparenten Solubilisierung notwendigen Konzentrationen der Solubilisator-Benetzmittel-Zubereitungen wiedergegeben, sowie die relative Erniedrigung der Konzentration im Verhältnis zum Solubilisator allein. Es zeigte sich, dass diese Verbesserung in einer signifikanten Verringerung der zur transparenten Solubilisierung notwendigen Konzentration des Solubilisators besteht. Dadurch konnten die geruchlichen Einflüsse des Solubilisators auf das Solubilisat deutlich reduziert und dessen Verwendbarkeit erweitert werden.

In vielen Fällen konnten des Weiteren transparente Solubilisate erst durch Zusatz der erfindungsgemäßen Benetzmittel hergestellt werden. Am signifikantesten waren die Verbesserungen bei den Mischungen der in EP1211258 B1 beschriebenen Alkylglycoside mit Polyglycerinestern und Acylglutamaten. Bei diesen Systemen konnte die zur transparenten Solubilisierung notwendige Menge Solubilisator mit Hilfe des Zusatzes von Benetzmitteln in einigen Fällen nahezu halbiert werden (6a und 6b).

**Tabelle 3: Ergebnisse der Solubilisierungsleistung der getesteten Solubilisator-Benetzmittel-Zubereitungen**

| | 1a | | 2a | | 3a | | 6a | | 6 b | |
|---|---|---|---|---|---|---|---|---|---|---|
| Aqua Power | 3,0 | - 14% | 3,0 | 0% | trü b | k.V . | 2,0 | - 33% | 1,8 | - 40% |
| Body Firm | 1,0 | 0% | 2,5 | 28% | 4,8 | -4% | 1,8 | 28% | 1,4 | 44% |
| Sugar Ray | 1,0 | 0% | 2,0 | 20% | 3,7 | -8% | 1,2 | 25% | 1,0 | 38% |
| Leafs in the trees | 0,5 | - 38% | 2,3 | -8% | 3,8 | -5% | 1,8 | 31% | 1,8 | 31% |
| Orange Ingwer | trüb | k.V . | 4,3 | - 10% | trü b | k.V . | 3,5 | - 30% | 3,4 | - 32% |
| Priceless Natura | 2,5 | - 12% | 3,3 | -6% | trü b | k.V . | 2,0 | - 43% | 2,0 | - 43% |
| Isoamyl Laurate | 6,5 | 7% | 9,0 | v.i . | 10 | v.i | 7,5 | v.i . | 7,5 | v.i . |
| PCA-Glyceryl Oleate | trüb | k.V . | trü b | k.V | 15 | v.i | 4,0 | - 11% | 4,0 | - 11% |
| Tocopherol (0,1%) | 1,3 | trü b | trü b | k.V | 3,0 | v.i | 1,5 | v.i . | 1,3 | v.i . |
| Bisabolol (0,1%) | 1,0 | 0,8 | 1,8 | 1,8 | 5,5 | v.i . | 1,0 | - 23% | 0,8 | - 38% |

| | | | | | | | | | | |
|---|---|---|---|---|---|---|---|---|---|---|
| k.V. = keine Verbesserung v.I. = vorher intransparent 1 95% PEG-40 Hydrogenated Castor Oil (Cremophor RH 40) a 5 % Glyceryl Caprylate (nicht erfindungsgemäß) 2 95% Cocoyl Proline; Aqua (Natisol) a 5% Glyceryl Caprylate (pH = 6,0) 3 95% Polyglyceryl-4 Caprylate, Decyl Glucoside, Sodium a Lauroyl Glutamate, Diglycerin, Aqua (Ressasol VPF) 5% Glyceryl Caprylate (pH> 7,0) 6 50% Caprylyl/Capric Wheat Bran / Straw Glycosides; a Aqua (Easysurf 6881) 25% Fusel Wheat Bran / Straw Glycosides; Aqua (Easysurf 6505) 10% Polyglyceryl-5 Oleate 10% Disodium Cocoyl Glutamate 5% Glyceryl Caprylate 6 50% Caprylyl/Capric Wheat Bran / Straw Glycosides; b Aqua (Easysurf 6881) 25% Fusel Wheat Bran / Straw Glycosides; Aqua (Easysurf 6505) 10% Polyglyceryl-5 Oleate 10% Disodium Cocoyl Glutamate 5% Caprylyl Glycol | | | | | | | | | | |

Zur erfindungsgemäßen Verwendung eines Benetzmittels wird dieses mit den Solubilisatorzusätzen und der zu solubiliserienden Substanz vor der Zugabe des Wassers homogen vermischt. Eine nachträgliche Zugabe des Benetzmittels in ein Solubilisat führt nicht zu dessen gewünschter Transparenz.

Tabelle 4 gibt eine Bewertung der Solubilisatorzusätze hinsichtlich der Erfüllung der erfindungsgemäßen Aufgabe wieder. Für alle getesteten Systeme wurde eine Verbesserung der der Solubilsierungsleistung und/oder der Anzahl solubilisierbarer Öle erzielt. Mit den Systemen 6a und 6 b wurde darüber hinaus PEG-freie, pH-unabhängige und vielseitige Solubilisatorzusätzen gefunden, welche die erfindungsgemäße Aufgabe in hervorragender Weise zu lösen im Stande sind und nahezu an die durchschnittliche Solubilisierungsleistung des PEG-40 hydrierten Rizinusöls heranreicht.

**Tabelle 4: Vergleichsübersicht über die getesteten Solubilisatorzusätze**

| Solubilisator | PEG-frei | durchschnittliche Solubilisierungsleistung | pH-Unabhängigkeit | Anzahl solubilisierbarer Öle |
|---|---|---|---|---|
| 1 | - | 0,9 | + | 9 / 10 |
| 1a | - | 0,8 | + | 8 / 10 |
| 2 | + | 2,8 | - | 8 / 10 |
| 2a | + | 2,3 | - | 8 / 10 |
| 3 | + | 4,3 | - | 3 / 10 |
| 3a | + | 4,1 | - | 7 / 10 |
| 6 | + | 2,2 | + | 8 / 10 |
| 6a | + | 1,6 | + | 10 / 10 |
| 6b | + | 1,4 | + | 10 / 10 |

## Patentansprüche

1. Solubilisatorzubereitung zur transparenten Solubilisierung von lipophilen Substanzen in wässrigen Medien enthaltend wenigstens eine Komponente ausgewählt aus der Gruppe bestehend aus:
a) Alkylpolyglycosiden der allgemeinen Formel R-O-(A)ⱼ-(B)ₖ-(C)ₗ-(D)ₘ-(E)ₙ, wobei R einen linearen oder verzweigten Alkylrest mit einer Kettenlänge von 4 - 12 Kohlenstoffatomen darstellt, A, B, C, D und E unabhängig voneinander Pentose- oder Hexose-Zuckereinheiten repräsentieren und j, k, l, m, n unabhängig voneinander Werte zwischen 0 und 1 annehmen können, wobei die Summe aller Werte j, k, l, m, n mindestens 1 beträgt;
b) Polyglycerinestern gebildet aus einer linearen oder verzweigten, gesättigten oder ungesättigten Fettsäure einer Kettenlänge von 8 - 20 Kohlenstoffatomen und einem Polymerisierungsgrad von n = 2 - 10;
c) N-acylierten Aminosäuren der allgemeinen Formel R'-CO-AS, wobei R' einen linearen oder verzweigten, gesättigten oder ungesättigten Alkylrest von 5 - 11 Kohlenstoffatomen und AS eine der in der Natur vorkommenden Aminosäuren, deren Racemat, Salze oder deren Ester repräsentiert;
wobei die Summe a) + b) + c) mindestens 50% der Gesamtzusammensetzung der Solubilisatorzubereitung ausmacht ;
**dadurch gekennzeichnet, dass** sie PEG-frei ist und zusätzlich enthält:
d) 3 - 10% mindestens eines Benetzmittel mit einem Molekulargewicht zwischen 100 und 250 g/mol und einem HLB-Wert zwischen 6 und 12, ausgewählt aus der Gruppe der Glycerinmonoester mit Fettsäuren einer Kettenlänge von n = 6 -10 Kohlenstoffatomen und der 1,2-Alkandiole mit einer Kettenlänge n = 6 - 10 Kohlenstoffatomen.

2. Solubilisatorzubereitung nach Anspruch 1, **dadurch gekennzeichnet, dass** das Alkylpolyglycosid vorwiegend aus den Pentosezuckereinheiten Xylose und/oder Arabinose aufgebaut ist.

3. Solubilisatorzubereitung nach Anspruch 1 oder 2, **dadurch gekennzeichnet, dass** der Polyglycerinester aus der Gruppe von Systemen, die einen HLB-Wert nach Griffin von 11 - 16 besitzen, ausgewählt ist, wobei besonders bevorzugt das Polyglycerin-5 Oleate ist.

4. Solubilisatorzubereitung nach einem der Ansprüche 1 bis 3, **dadurch gekennzeichnet, dass** die N-acylierte Aminosäure aus der Gruppe der Kokosfettsäureamide des Prolins oder der Glutaminsäure und den Salzen der vorgenannten Verbindungen ausgewählt ist, wobei besonders bevorzugt das Kokosfettsäureamid der Glutaminsäure ist.

5. Solubilisatorzubereitung nach einem der Ansprüche 1 bis 4, **dadurch gekennzeichnet, dass** das Benetzmittel ausgewählt ist aus der Gruppe bestehend aus Glyceryl Caprylate und Caprylyl Glycol.

6. Solubilisatorzubereitung nach einem der Ansprüche 1 bis 5, **dadurch gekennzeichnet, dass** sie enthält:
| | |
|---|---|
| 30 - 40 % | Caprylyl/Capric Wheat Bran / Straw Glycosides |
| 10 - 20 % | Fusel Wheat Bran / Straw Glycosides |
| 5 - 15 % | Polyglyceryl-5 Oleate |
| 5 - 15 % | Disodium Cocoyl Glutamate |
| 3 - 10% | Glyceryl Caprylate |

7. Solubilisatorzubereitung nach einem der Ansprüche 1 bis 5, **dadurch gekennzeichnet, dass** sie enthält:
| | |
|---|---|
| 30 - 40 % | Caprylyl/Capric Wheat Bran / Straw Glycosides |
| 10 - 20 % | Fusel Wheat Bran / Straw Glycosides |
| 5 - 15 % | Polyglyceryl-5 Oleate |
| 5 - 15 % | Disodium Cocoyl Glutamate |
| 3 - 10% | Caprylyl Glycol |

8. Verfahren zur Herstellung eines Solubilisats, **gekennzeichnet durch** die Schritte des homogenen Verrührens der zu solubilisierenden, lipophilen Komponenten mit einer Solubilisatorzubereitung nach einem der Ansprüche 1 bis 7 und anschließendem Verdünnen der Mischung mit einer wässrigen Phase.

9. Verwendung eines Solubilisates hergestellt nach dem Verfahren des Anspruchs 8 in einem Produkt zur Reingung, Beduftung, Desodorierung oder Pflege der Haut, der Schleimhaut und/oder der Haare.

## Claims

1. Solubilizer preparation for the transparent solubilization of lipophilic substances in aqueous media containing at least one component selected from the group consisting of:
a) alkylpolyglycosides of the general formula R-O-(A)ⱼ-(B)ₖ-(C)ₗ-(D)ₘ-(E)ₙ, in which R represents a linear or branched alkyl radical having a chain length of 4-12 carbon atoms, A, B, C, D and E independently represent pentose or hexose sugar units, and j, k, 1, m and n are able to independently have values between 0 and 1, the sum of all the values j, k, 1, m and n equalling at least 1;
b) polyglycerol esters formed of a linear or branched, saturated or unsaturated fatty acid having a chain length of 8-20 carbon atoms and a degree of polymerisation of n = 2-10;
c) N-acylated amino acids of the general formula R'-CO-AS, in which R' represents a linear or branched, saturated or unsaturated alkyl radical having 5-11 carbon atoms and AS represents a naturally occurring amino acid, the racemate, salts or esters thereof; the sum a) + b) + c) constituting at least 50 % of the overall composition of the solubilizer preparation;
**characterised in that** it is PEG-free and additionally contains:
d) 3-10 % of at least one wetting agent having a molecular weight of between 100 and 250 g/mol and a HLB value of between 6 and 12, selected from the group of glycerol monoesters comprising fatty acids having a chain length of n = 6-10 carbon atoms and of 1,2-alkanediols having a chain length of n = 6-10 carbon atoms.

2. Solubilizer preparation according to claim 1, **characterised in that** the alkyl polyglucoside is formed predominantly of the pentose sugar units xylose and/or arabinose.

3. Solubilizer preparation according to either claim 1 or claim 2, **characterised in that** the polyglycerol ester is selected from the group of systems which have an HLB value according to Griffin of 11-16, polyglyceryl-5 Oleate being particularly preferred.

4. Solubilizer preparation according to any of claims 1 to 3, **characterised in that** the N-acylated amino acid is selected from the group of coconut fatty acid amides of prolines or of glutamic acid and the salts of the abovementioned compounds, the coconut fatty acid amide of glutamic acid being particularly preferred.

5. Solubilizer preparation according to any of claims 1 to 4, **characterised in that** the wetting agent is selected from the group consisting of Glyceryl Caprylate and Caprylyl Glycol.

6. Solubilizer preparation according to any of claims 1 to 5, **characterised in that** it contains:
30 - 40 %Caprylyl/Capric Wheat Bran / Straw Glycosides
10 - 20 %Fusel Wheat Bran / Straw Glycosides
5 - 15 % Polyglyceryl-5 Oleate
5 - 15 % Disodium Cocoyl Glutamate
3 - 10 % Glyceryl Caprylate

7. Solubilizer preparation according to any of claims 1 to 5, **characterised in that** it contains:
30 - 40 %Caprylyl/Capric Wheat Bran / Straw Glycosides
10 - 20 %Fusel Wheat Bran / Straw Glycosides
5 - 15 % Polyglyceryl-5 Oleate
5 - 15 % Disodium Cocoyl Glutamate
3 - 10 % Caprylyl Glycol

8. Method for producing a solubilizate, **characterised by** the steps of homogenously mixing the lipophilic components to be solubilized with a solubilizer preparation according to any of claims 1 to 7 and subsequent diluting of the mixture with an aqueous phase.

9. Use of a solubilizate produced according to the method of claim 8 in a product for cleaning, fragrancing, deodorisation or care of the skin, mucous membrane and/or hair.

## Revendications

1. Préparation solubilisatrice pour la solubilisation transparente de substances lipophiles dans des milieux aqueux, contenant au moins un composant choisi dans le groupe consistant en :
a) les alkylpolyglycosides de formule générale R-O-(A)ⱼ-(B)ₖ-(C)ₗ-(D)ₘ-(E)ₙ, dans laquelle R représente un radical alkyle à chaîne droite ou ramifiée ayant une longueur de chaîne de 4 à 12 atomes de carbone, A, B, C, D et E représentent chacun indépendamment des autres une unité sucre pentose ou hexose, et j, k, 1, m, n peuvent chacun indépendamment des autres prendre une valeur comprise entre 0 et 1, la somme de toutes les valeurs j, k, 1, m, n étant d'au moins 1 ;
b) les esters du polyglycérol, formés à partir d'un acide gras saturé ou insaturé à chaîne droite ou ramifiée, ayant une longueur de chaîne de 8 à 20 atomes de carbone, et ayant un degré de polymérisation n = 2 - 10 ;
c) les acides aminés N-acylés de formule générale R'-CO-AS, dans laquelle R' représente un radical alkyle saturé insaturé à chaîne droite ou ramifiée, ayant 5 à 11 atomes de carbone, et AS représente l'un des acides aminés naturels, leur racémate, leurs sels ou esters ;
la somme a) + b) + c) représentant au moins 50 % de la composition totale de la préparation solubilisatrice ;
**caractérisée en ce qu'**elle est exempte de PEG, et qu'elle contient en outre !
d) 3 à 10 % d'au moins un agent mouillant ayant une masse moléculaire comprise entre 100 et 250 g/mole et une valeur HLB comprise entre 6 et 12, choisi dans le groupe des monoesters du glycérol et d'acides gras ayant une longueur de chaîne n = 6-10 atomes de carbone, et des 1,2-alcanediols ayant une longueur de chaîne n = 6-10 atomes de carbone.

2. Préparation solubilisatrice selon la revendication 1, **caractérisée en ce que** l'alkylpolyglycoside est pour l'essentiel constitué des unités de sucre pentose que sont le xylose et/ou l'arabinose.

3. Préparation solubilisatrice selon la revendication 1 ou 2, **caractérisée en ce que** l'ester du polyglycérol est choisi dans le groupe de systèmes qui ont une valeur HLB selon Griffin de 11 à 16, l'oléate de polyglycérol-5 étant particulièrement préféré.

4. Préparation solubilisatrice selon l'une des revendications 1 à 3, **caractérisée en ce que** l'acide aminé N-acylé est choisi dans le groupe des esters d'acides gras dérivés de l'huile de coprah de la proline ou de l'acide glutamique, et les sels des composés mentionnés ci-dessus, l'amide d'acides gras de l'huile de coprah de l'acide glutamique étant particulièrement préféré.

5. Préparation solubilisatrice selon l'une des revendications 1 à 4, **caractérisée en ce que** l'agent mouillant est choisi dans le groupe consistant en le caprylate de glycéryle et le caprylylglycol.

6. Préparation solubilisatrice selon l'une des revendications 1 à 5, **caractérisée en ce qu'**elle contient :
30-40 % de Caprylyl/Capryl Wheat Bran/Straw Glycosides
10-20 % de Fusel Wheat Bran/Straw Glycosides
5-15 % de Polyglyceryl-5 Oleate
5-15 % de Disodium Cocoyl Glutamate
3-10 % de Glyceryl Caprylate

7. Préparation solubilisatrice selon l'une des revendications 1 à 5, **caractérisée en ce qu'**elle contient :
30-40 % de Caprylyl/Capryl Wheat Bran/Straw Glycosides
10-20 % de Fusel Wheat Bran/Straw Glycosides
5-15 % de Polyglyceryl-5 Oleate
5-15 % de Disodium Cocoyl Glutamate
3-10 % de Caprylyl Glycol

8. Procédé de fabrication d'un solubilisat, **caractérisé par** les étapes de délayage jusqu'à homogénéité des composants lipophiles à solubiliser avec une préparation solubilisatrice selon l'une des revendications 1 à 7, puis dilution du mélange avec une phase aqueuse.

9. Utilisation d'un solubilisat fabriqué par le procédé selon la revendication 8 dans un produit destiné au nettoyage, au parfumage, à la désodorisation ou aux soins de la peau, des muqueuses et/ou des cheveux.
